# EUROPEAN PATENT APPLICATION

(11) **EP 1 060 742 A1**
(43) Date of publication of application: **20.12.2000**
(21) Application number: 99906473.6
(22) Date of filing: 25.02.1999
(51) Int. Cl.: A61K 31/435

(54) **INTERSTITIAL ADENOSINE LEVEL-ELEVATING AGENTS**

(30) Priority: 27.02.1998 JP 6203398
(71) Applicant: Wyeth Lederle Japan LTD., Chuo-ku, Tokyo 104-0031 (JP)
(72) Inventor: SHIBA, Toshiharu Room 506, Tokyo 111-0034 (JP); SHIKATA, Yoshiyuki, Asaka-shi Saitama 351-0025 (JP)
(74) Representative: Weisert, Annekäte, Dipl.-Ing. Dr.-Ing.
(86) International application number: JP9900855
(87) International publication number: WO9943321

(57) **Abstract**

Tissue adenosine level-elevating agents and therapeutic agents for treating heart failures containing as an active ingredient 2,5-diphenyl-7-hydroxy-6-alkoxy-carbonyl-8-(2-pyridyl)acetyl-1,9-diazabicyclo[4.3.0]nona-3,8-dien of the following formula (I): 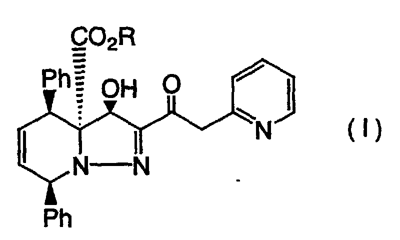 or pharmacologically acceptable salts thereof, in particular, the above drugs containing as an active ingredient, the compound of formula (I), wherein R is methyl, or pharmacologically acceptable salts thereof.

## Description

### TECHNICAL FIELD

The present invention relates to tissue adenosine level-elevating agents, and more particularly, to tissue adenosine level-elevating agents containing 1,9-diazabicyclo[4.3.0]nonane derivatives or pharmacologically acceptable salts thereof as active ingredient. The invention further relates to therapeutic agents for treating heart failure disease, which contain the same compound or pharmacologically acceptable salts thereof as an active ingredient.

### BACKGROUND ART

Adenosine, (6-amino-9-β-D-riboflanosyl-9H-purine), which is purine nucleotide and a structural component of nucleic acids or co-enzymes, is known to be involved in important physiological reactions *in vivo* system. Adenosine, for example, exhibits various pharmacological effects that are of clinical importance including analgesic, sedative, hypnotic, anxiolytic, anticonvulsive and muscle relaxant effects.

Accordingly, development of compounds that can elevate tissue adenosine level may lead to development of functional substances that are useful as analgesics, sedatives, hypnotics, anxiolytics, anticonvulsives and muscle relaxants.

The present inventors have proposed heretofore novel 1,9-diazabicyclo[4,3,0]nonan derivatives that exhibit protective effects for a heart from failures in the cardiovascular systems caused by ischemia, anoxia or hypoxia (See, WO97/06168).

These compounds have proven to be effective in preventing damage to tissue when the blood flow is blocked in a brain or heart in the event of cerebral infarction, cardiac infarction or angina pectoris and in facilitating the recovery of the cardiovascular function. Specifically, it has been shown that the compounds have a remarkable myocardial protective effect during reperfusion of ischemia.

In the course of study, the present inventors have found that these 1,9-diazabicyclo[4.3.0]nonan derivatives cause the tissue adenosine level to rise and that it is this effect of increasing the tissue adenosine level which contributes to the myocardial protective effect during reperfusion of ischemia. Because of their specific effect of increasing the tissue adenosine level, it is expected, in view of the above discussion, that these compounds not only exhibit effects on cardiovascular systems, e.g., the myocardial protective effect during reperfusion of ischemia, but also have various other physiological effects such as analgesic, sedative, hypnotic, anxiolytic, anticonvulsive and muscle relaxant effects.

Among other various pharmacological effects, the present inventors' further study was focused on the effect of the 1,9-diazabicyclo[4.3.0]nonan derivatives to facilitate the recovery of the cardiovascular function, and has revealed that these compounds, in addition to having the myocardial protective effect during reperfusion of ischemia, are capable of suppressing hypercardia in heart failure models. Further, they are effective in suppressing various myocardiopathies such as myocardial necrosis and myofibrosis cordis and, therefore, are useful as medicaments for ischemic heart disorders as well as for heart failures.

In view of the present state described above, it is an objective of the present invention to provide a tissue adenosine level-elevating agent containing novel 1,9-diazabicyclo[4.3.0]nonan derivatives as an active ingredient and to provide a therapeutic agent for treating heart failures.

### DISCLOSURE OF THE INVENTION

To achieve the aforementioned objectives, one aspect of the present invention provides tissue adenosine level-elevating agents containing, as an active ingredient, 2,5-diphenyl-7-hydroxy-6-alkoxycarbonyl-8-(2-pyridyl)acetyl-1,9-diazabicyclo[4.3.0]nona-3,8-dien represented by the following formula (I): 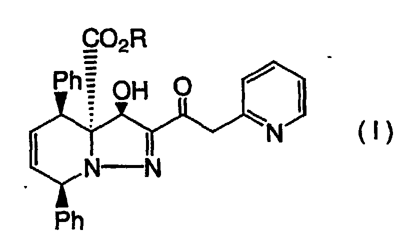 wherein R is a lower alkyl group; or pharmacologically acceptable salts thereof.

Another aspect of the present invention provides therapeutic agents for treating heart failure containing as an active ingredient 2,5-diphenyl-7-hydroxy-6-alkoxy-carbonyl-8-(2-pyridyl)acetyl-1,9-diazabicyclo[4.3.0]nona-3, 8-dien of the formula (I).

### BRIEF DESCRIPTION OF THE DRAWINGS

Fig.1 is a graph showing the results of the pharmacological test example 1 described later in which tissue adenosine level is increased.

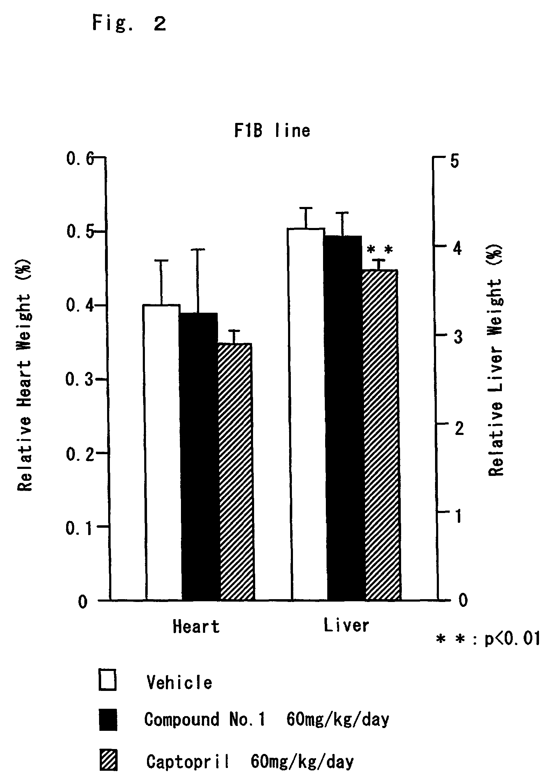

Fig 2 is a graph showing the results of the pharmacological test example 2 described later in which the effects on the relative heart weight and relative liver weight were examined in F1B hamsters.

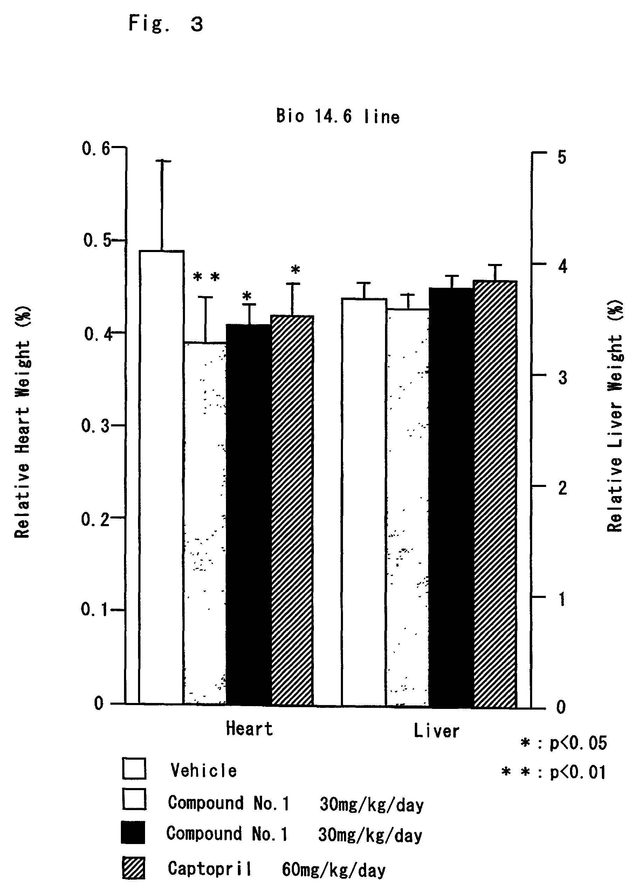

Fig.3 is a graph showing the results of the pharmacological test example 2 described later in which the effects on the relative heart weight and relative liver weight were examined in Bio 14.6 hamsters.
Fig.4 is a graph showing the results of the pharmacological test example 2 described later in which plasma NE level (45 days after the administration) was examined.
Fig.5 is a graph showing the results of the pharmacological test example 2 described later in which plasma NE level (88 days after the administration) was examined.

### BEST MODES FOR CARRYING OUT THE INVENTION

In the compound of the formula (I) as the active ingredient of the present invention, the lower alkyl, which is the substituent R, refers to straight or branched chain alkyl groups having about 1 to 4 carbon atoms. Examples thereof include methyl, ethyl, n-propyl, isopropyl, n-butyl, isobutyl, *sec*-butyl, *tert*-butyl, or the like.

The particularly preferred effect on increasing the tissue adenosine level and the therapeutic effect for heart failures has been observed when R is methyl.

Accordingly, a more specific embodiment of the present invention provides tissue adenosine level-elevating agents and therapeutic agents for treating heart failures containing as an active ingredient 2,5-diphenyl-7-hydroxy-6-methoxycarbonyl-8-(2-pyridyl)acetyl-1,9-diazabicyclo[4.3.0]-nona-3,8-dien, which is a compound with R being methyl in the formula (I), or pharmacologically acceptable salts thereof.

With regard to the therapeutic agents provided by the present invention, 1,9-diazabicyclo[4.3.0]nonan derivatives, which serve as the active ingredient and are represented by the formula (I), have already been proposed by the present inventors (WO97/06168) and can be manufactured by the methods described in the specification thereof.

Among these derivatives, 2,5-diphenyl-7-hydroxy-6-methoxycarbonyl-8-(2-pyridyl)acetyl-1,9-diazabicyclo[4.3.0]-nona-3,8-dien, which is referred to as compound No.1 hereinafter, is particularly effective. Specifically, the compound has the following physicochemical properties: ¹H-NMR(CDCl₃) δ: 3.71 (s,3H), 4.19 (s,1H), 4.54-4.60 (m,1H), 5.22 (s,1H), 5.66-5.67 (m,1H), 5.84-5.99 (m,2H), 6.87-7.63 (m,13H), 8.42 (d,1H, J=4.0 Hz)

The compounds represented by the formula (I), which are the active ingredient of the present invention, may be used in the form of pharmacologically acceptable acid salts obtained by using an organic or inorganic acid. Examples of the organic acid include: lower fatty acids such as formic acid, acetic acid, propionic acid, butyric acid, trifluoroacetic acid and trichloroacetic acid; substituted or unsubstituted benzoic acids such as benzoic acid and p-nitro benzoic acid; (halo)lower alkyl sulfonic acids such as methanesulfonic acid, trifluoromethanesulfonic acid; substituted or unsubstituted arylsulfonic acids such as benzenesulfonic acid, p-nitrobenzenesulfonic acid, p-bromobenzenesulfonic acid, toluenesulfonic acid, 2,4,6-triisopropylbenzenesulfonic acid; and organic phosphoric acids such as diphenylphosphoric acid.

Examples of the inorganic acid include hydrochloric acid, sulfuric acid, hydrobromic acid, hydriodic acid, borofluoric acid, perchloric acid, nitrous acid and the like.

Specifically, the therapeutic agents of the present invention containing the compounds of formula (I) as an active ingredient may be orally administered to a human in the form of a pharmacologically acceptable composition containing an effective amount of the active ingredient. The dosage may vary depending on age, weight or conditions of the subject. Preferably, a dosage may be 10 to 1000mg of the agent orally administered at a time or in two to three times a day.

Preferred dosage forms for oral administration include tablets, capsules, powders, troches and solutions. These formulations can be manufactured by methods known to those skilled in the art. For example, the compounds of formula (I) may be formed into a preparation such as tablets, capsules, powders, granules or troches by properly admixing the compound with an excipient such as starch, mannitol and lactose; a binder such as sodium carboxymethyl cellulose and hydroxypropyl cellulose; a disintegrating agent such as crystallized cellulose and calcium carboxymethyl cellulose; a lubricant such as talc and magnesium stearate; and an fluidity enhancing agent such as light anhydrous silicate.

Also, the compounds of formula (I) in accordance with the present invention may be administered as an injection. Examples of such preparations include injections that may be prepared by preliminarily dissolving or dispersing the compound into saline with the aid of typical surfactants or dispersing agents, crystallized preparations for injection, or freeze-dried and powdered preparations. These preparations may contain typical pH conditioners or stabilizers.

The range of dose and route of administration of the injections are not limited to a specific dose range or route and may vary depending on age, weight or conditions of the subjects. The injections may be administered intravenously, intraarterialy, or subcutaneously and may be administered by a bolus injection or by a drip.

### Examples

The present invention will now be described in further detail by presenting the results of pharmacological tests to show the specific effects of increasing the tissue adenosine level as well as the anti-heart failure activity and myocardiopathy suppressing activity of the present invention and by presenting specific examples of formulations of the present invention.

### Pharmacological Test Example 1: Effects on myocardiac adenosine level in extirpated hearts of rat.

### 1. Methods:

Male Sprange-Dawley rats aged 7 to 8 weeks were used. Hearts were extirpated under anesthesia by pentobarbital (50mg/kg, intra-abdominally injected) and were suspended in a Langendorff's apparatus. A Krebs-Henseleit solution (K-H solution: 118mM NaCl; 4.7mM KCl; 2.55mM CaCl₂; 1.18mM MgSO₄; 1.18mM KH₂PO₄; 24.88mM NaHCO₃; 11.1mM glucose; mixture of 5% CO₂ + 95% O₂ passed, pH=7.4) was perfused from the aorta in a retrograde manner under a constant pressure of 75mmHg.

The samples were allowed to stabilize for 15 minutes and were then perfused for 15 minutes with a K-H solution added with compound No.1 as a test compound. The hearts were then frozen with liquid nitrogen. 10 equivalents of 0.6N perchloric acid were added to the frozen tissue and the tissue was homogenized and neutralized by 1N KOH and was then centrifuged. After centrifugation, the level of adenosine in the supernatant was measured by HPLC.

In making the compound No.1 solution, the compound No.1 was first dissolved in 100% DMSO and then added to the K-H solution to a final concentration of 0.04%.

Propranol was used as a control compound for the test and 0.04% DMSO solution was used as a vehicle control.

Measurements were expressed in mean ± S.E., and tests for significant differences between the respective groups were performed using Dunnett's multiple comparison test.

### 2. Results

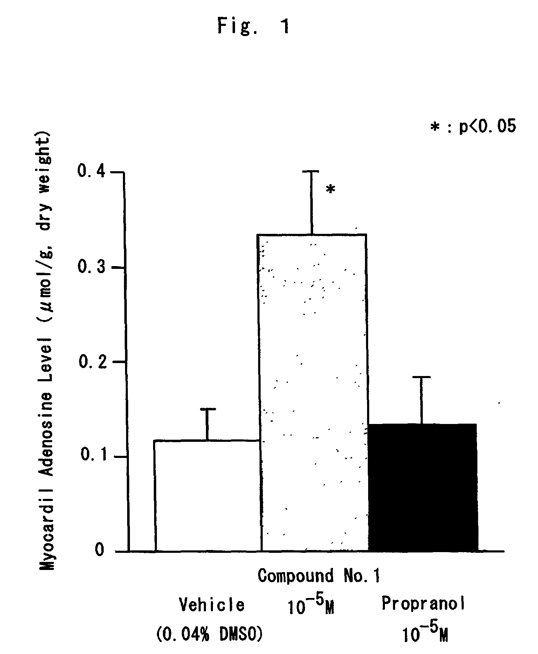

The results are shown in Fig.1.

As apparent from the results shown in Fig.1, the myocardial adenosine level was increased three-fold as compared to the vehicle control group after the 15-minute perfusion of 10⁻⁵ M of compound No.1. In contrast, no significant difference was observed in the myocardial adenosine level between the group in which 10⁻⁵ M of propranol as a control compound was perfused for 15 minutes and the vehicle control group.

Accordingly, it is demonstrated that the compound No.1, which is the active ingredient of the present invention, has a specific effect to increase the tissue adenosine level.

### Pharmacological Test Example 2: Effects on heart failure and myocardiopathy

### 1. Methods:

### (1) Test animals and feeding conditions:

Male cardiomyopathic Syrian hamsters (Bio 14.6 strain) aged 7 weeks were used. Male aged-matched normal healthy golden hamsters (F1B strain) were used as control animals. These hamsters were purchased from Biobreeders Inc. Throughout the test period, the animals were housed in stainless steel cage in a temperature (23 ± 1C° )controlled room where a daily light-dark cycle was maintained artificially at 12 hours intervals. The animals were fed with commercially available pellet (CE-2, CLEA Japan, Inc.) and provided *adde libitum* with feed pellets and water. Incidentally, after they were purchased, the animals were acclimated to the above environment at least one week before subjected to experiments.

### (2) Test compounds and methods of administration

Compound No.1 was used as a test compound. The test compound was suspended in a 0.5% sodium carboxymethyl cellulose (CMC-Na) solution. This dosing solution was orally administered for 87 days at dosages of 30mg/kg/day and 60mg/kg/day and a dose volume of 20ml/kg.

Captopril was used as a control compound. The compound was similarly suspended in a 0.5% CMC-Na solution and was orally administered for 87 days at a dosage of 60mg/kg/day and a dose volume of 20ml/kg.

A group of animals administered with a 0.5% CMC-Na solution served as a vehicle control group. The solution was administered once daily from Monday through Friday but not on Saturdays and Sundays.

### (3) Measurements

Body weight was recorded on every Monday and Thursday after the administration of the test compound. Blood samples were also collected 30, 60, and 80 days after administration, and the plasma samples were examined for clinical chemistry parameters.

Blood samples were collected before administration of the test compound, and 45 and 88 days after administration. The blood samples were collected from venous plexus of eyegrounds under anesthesia by ether. Blood plasma was separated from the samples and plasma norepinephrine (NE) levels were measured using a catecholamine assay system.

### (4) Myocardial tissue analysis

Myocardial tissue preparations were prepared 88 days after the administration was started. The animals were sacrificed by exsanguination under anesthesia by ether. Chests were opened and hearts extirpated. Weight of the hearts was measured and the hearts were fixed in a 10% buffered neutral formaldehyde solution. Bases of the hearts were sliced to a 3mm thickness and formed into paraffin slices with respect to the side of the base using ordinary methods. The slices were stained with kossa, hematoxylin/eosin (H&E) and H&E/aniline blue stains. The preparations of the hearts so prepared were measured in area for regions of cardiac fibrosis, mineral deposition, myocardial necrosis, and macrophage infiltration, using an image analyzing system (Mac SCOPE Ver.2.1, Mitsuya Shoji).

Measurements were expressed in mean ± S.E., and tests for significant differences between the respective groups were performed using Dunnett's multiple comparison test.

### 2. Results

### (1) Effects on body weight change and heart weight

Each dosage of the test compound did not affect the changes in body weight in Bio 14.6 and F1B hamsters.

With respect to the relative heart weight and relative liver weight on day 88, a 20% increase was observed in the relative heart weight in Bio 14.6 hamsters as compared to F1B hamsters, indicative of an increase in heart weight due to hypertrophic cardiomyopathy. While the compound No.1 did not affect the relative heart weight in F1B hamsters, a significant suppression of increase in relative heart weight was seen in Bio 14.6 hamsters expressing cardiomyopathy in both dosage groups of 30mg/kg/day and 60mg/kg/day. The relative heart weight of Bio 14.6 hamsters in these groups was substantially the same as that of the F1B line. The same results were obtained for the groups administered 60mg/kg/day of captopril as a control compound.

With respect to liver weight, the test compound No.1 did not have effects on relative liver weight of either Bio 14.6 or F1B hamsters, whereas a significant decrease in relative liver weight was observed in F1B hamsters in the group administered with captopril.

The results are shown in Tables 2 and 3.

### (2) Effects on plasma NE levels and clinical chemistry parameters

When an excess load applies on a heart as in the case of hypercardia, norepinephrine (NE) is released from the terminals of the sympathetic nerves as compensatory response, which in turn enhances the contraction of the heart. Accordingly, it is known that the plasma NE level in Bio 14.6 hamsters, which serve as a model expressing cardiomyopathy, is higher than that in F1B hamsters.

45 days after administration of the vehicle was started, a higher plasma NE level was observed in Bio 14.6 hamsters as compared to F1B hamsters. The difference was insignificant, however.

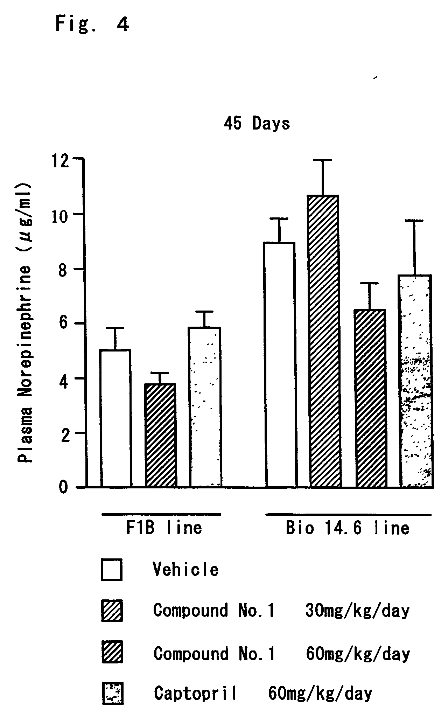

Each dosage of the test compound did not have significant effects on the plasma NE level when compared to the vehicle control groups. The results are shown in Fig.4.

88 days after administration of the vehicle, a significantly higher plasma NE level was observed in Bio 14.6 hamsters as compared to F1B hamsters. Administration of the test compound had no effect on the plasma NE level in F1B hamsters.

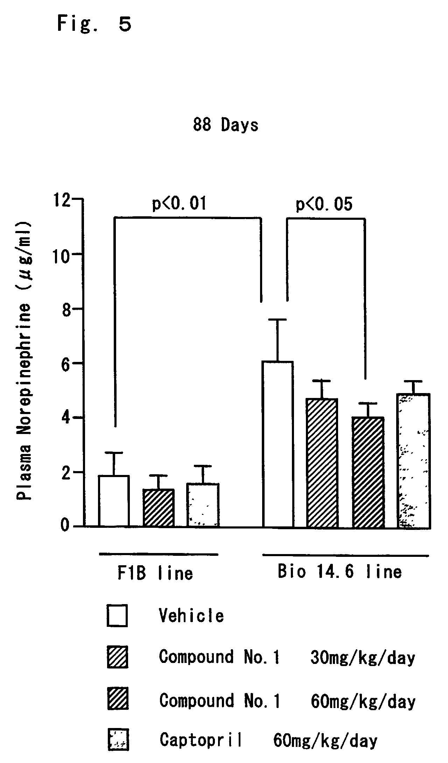

In Bio 14.6 hamsters, the compound No.1 caused the plasma NE level to decrease in a dose-dependent manner. A significantly lower level was observed in the group administered by 60mg/kg/day as compared to the vehicle control group. The same results were obtained for the group administered by 60mg/kg/day of captopril. These results are shown in Fig.5.

Of the clinical chemistry tests performed 30, 60, and 88 days after the administration was started, only the ones performed on day 30 on the groups of Bio 14.6 animals that were administered 60mg/kg/day of compound No.1 or captopril showed the tendency of decrease AST (GOT) and LDH levels. All the other tests including those performed on F1B animals showed no effect by the administration of the test compound.

### (3) Histological evaluation of myocardial tissue preparation

The degree of myocardiopathy was examined by the image analysis of the heart muscle tissue preparations that were prepared 88 days after administration of the test compound. Little tissue damage was found in F1B hamsters.

In the vehicle control group of the Bio 14.6, myocardiopathy such as myocardial necrosis, macrophage infiltration, fibrosis, and mineral deposition were found. After the compound No.1 was administered to the Bio 14.6 animals, a significant decrease in area was observed in the regions of myocardial necrosis, macrophage infiltration and fibrosis, as well as in area of cardiac legions, which is the sum of the areas of the first three regions, in the group administered 30mg/kg/day of the compound No.1. A significant decrease in area was also observed in the regions of myocardial necrosis and macrophage infiltration in the group administered 60mg/kg/day.

In the group administered 60mg/kg/day of captopril as a control compound, a significant decrease in area was also observed in the regions of myocardial necrosis, macrophage infiltration, and fibrosis as well as in area of cardiac lesions.

These results suggest that the compounds of the present invention have specific therapeutic effects for heart failures. Their efficacy is equivalent to, or exceeds, that of captopril that is already in clinical use.

### Pharmacological Test Example 3: Toxicity tests

10 male ICR mice weighing 20-23g were subcutaneously administered with a 10% aqueous polyethylene glycol 400 solution of the compound No.1 and were observed for one week. There was no mortality and no clinical observations related to treatment with the compound No.1 when administered 10ml/kg of the solution.

Similarly, a 0.025N hydrochloric acid-saline solution containing the compound No.1 was orally administered to mice after 16-hour fasting period, and overall behavior was observed for two weeks following the administration. LD₅₀ was determined using the Richfield-Wilcockson method.

As a result, there was no mortality and no clinical observations. The LD₅₀ for the compound No.1 was over 100mg/kg.

### Examples of formulations

### (1) Tablets

| | |
|---|---|
| Composition: compound No.1 | 15g |
| Lactose | 120g |
| fine crystal cellulose | 25g |
| corn starch | 25g |
| 5% aqueous hydroxypropylmethyl cellulose solution | 100ml |
| magnesium stearate | 5g |

Using ordinary methods, the above components were processed through mixing, granulation and drying and were then formed into tablets, each of which had weight of 190mg and contained 15mg of the compound No.1 as the active ingredient per one tablet.

### (2) Injections

12.5mg of the compound No.1 in the form of powder were placed in a vial. Upon use, about 3-4ml of diluted water for injection were added to dissolve the compound in order to make an injection.

### INDUSTRIASL APPLICABILITY

As discussed in detail hereinabove, 1,9-diazabicyclo-[4.3.0]nonan derivatives of the formula (I) or pharmacologically acceptable salts thereof, in particular, 2,5-diphenyl-7-hydroxy-6-methoxycarbonyl-8-(2-pyridyl)acetyl-1,9-diazabicyclo[4,3,0]nona-3,8-dien or pharmacologically acceptable salts thereof have a notable effect of increasing tissue adenosine level. Accordingly, these compounds are useful functional substances as analgesics, sedatives, hypnotics, anxiolytics, anticonvulsives, muscle relaxants and cardiovascular therapeutic agents. Further, the compounds of the present invention have proven to have a notable efficacy as a therapeutic agent for heart failures and their potential in medical application is remarkable.

## Claims

1. A tissue adenosine level-elevating agent containing as an active ingredient 2,5-diphenyl-7-hydroxy-6-alkoxycarbonyl-8-(2-pyridyl)acetyl-1,9-diazabicyclo[4.3.0]-nona-3,8-dien of the following formula (I): 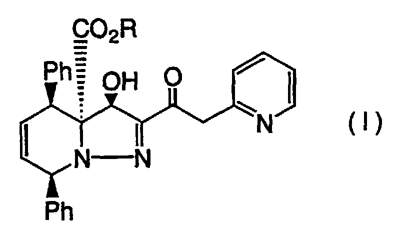 wherein R is a lower alkyl group; or pharmacologically acceptable salts thereof.

2. A tissue adenosine level-elevating agent containing as an active ingredient 2,5-diphenyl-7-hydroxy-6-methoxycarbonyl-8-(2-pyridyl)acetyl-1,9-diazabicyclo[4.3.0]-nona-3,8-dien or pharmacologically acceptable salts thereof.

3. A therapeutic agent for treating heart failure containing as an active ingredient the 2,5-diphenyl-7-hydroxy-6-alkoxycarbonyl-8-(2-pyridyl)acetyl-1,9-diazabi-bicyclo[4.3.0]nona-3,8-dien of the formula (I) or pharmacologically acceptable salts thereof as recited in claim 1.

4. A therapeutic agent for treating heart failure containing as an active ingredient 2,5-diphenyl-7-hydroxy-6-methoxycarbonyl-8-(2-pyridyl)acetyl-1,9-diazabicyclo[4.3.0]-nona-3,8-dien or pharmacologically acceptable salts thereof.
